# FASCICULE DE BREVET EUROPEEN

(11) **EP 0 665 752 B1**
(45) Date de publication et mention de la délivrance du brevet: **04.06.2003**
(21) Numéro de dépôt: 93924096.6
(22) Date de dépôt: 19.10.1993
(51) Int. Cl.: A61K 31/566, A61K 31/57, A61P 5/46

(54) **COMPOSITIONS PHARMACEUTIQUES CONTENANT DES DERIVES DE STEROIDES NATURELS 3BETA HYDROXYLES ET LEUR UTILISATION**
3-beta-hydroxylierte natürliche Steroidderivate enthaltende Arzneimittelzusammensetzungen und ihre Verwendung
PHARMACEUTICAL COMPOSITIONS CONTAINING 3-BETA-HYDROXYLATED NATURAL STEROID DERIVATIVES, AND USE THEREOF

(30) Priorité: 20.10.1992 FR 9212548
(43) Date de publication de la demande: 09.08.1995
(73) Titulaire: LABORATOIRES MAYOLY SPINDLER, 78401 Chatou Cédex (FR)
(72) Inventeur: MORFIN, Robert, F-75013 Paris (FR)
(74) Mandataire: Corizzi, Valérie
(86) Numéro de dépôt international: FR9301029
(87) Numéro de publication internationale: WO94008588

(56) Documents cités:
- WO-A-91/04030
- WO-A-92/03925
- WO-A-93/20696
- WO-A-94/03176
- DE-A- 3 812 595
- US-A- 4 628 052
- US-A- 4 898 694
- US-A- 5 077 284

## Description

La présente invention est relative à des compositions pharmaceutiques contenant à titre de principe actif des dérivés 7-hydroxylés d'hormones stéroïdes naturelles possédant, le cas échéant, une fonction 3β hydroxyle, et leur utilisation comme déclencheur ou stimulateur de l'immunité (ci-après dénommé "effecteur de l'immunité") et plus particulièrement de l'immunité cellulaire ; ces compositions pharmaceutiques peuvent être également utilisées comme agents anti-glucocorticoïdes.

La famille des stéroïdes hormonaux 3β-hydroxylés comprend: la pregnènolone connue comme 3β-hydroxy-5-pregnène-20-one (désignée ci-après par PREG), la déhydroépiandrostérone connue comme 3β-hydroxy-5-androstène-17-one (désignée ci-après par DHEA), l'androstènediol connu comme 5-androstène-3β,17β-diol (désigné ci-après par 5-DIOL), l'iosandrostérone ou épiandrostérone connue comme 3β-hydroxy-5α-androstane-17-one (désignée ci-après par ISOA) et l'androstanediol connu comme 5α-androstane-3β, 17β-diol (désigné ci-après par ADIOL).

La PREG est connue comme étant le précurseur de l'ensemble des hormones stéroïdes. La PREG est formée irréversiblement à partir du cholestérol dans des tissus et des organes comme la partie corticale des glandes surrénales, les gonades (E. Baulieu, Hormones, Publ. Hermann (1978)) et le cerveau (Z. Hu et al., Proc. Nat. Acad. Sci. USA, 84, 8215-8219 (1987)). Formée dès le plus jeune âge, ses quantités circulantes sont élevées et relativement stables (E. DePeretti et E. Mappus, J. Clin. Endocr. Metab., 57, 550-556 (1983)). Elle peut être transformée en progestérone même dans le cerveau (Y. Akwa et al., J. Cell. Biol., 121, 135-143 (1993)) mais sa transformation irréversible en hormones minéralocorticoïdes et glucocorticoïdes n'a lieu que dans les glandes corticosurrénales (E. Baulieu, Hormones, Publ. Hermann (1978)).

La DHEA est un 17-cétostéroïde qui est quantitativement une des hormones stéroïdes adrénocorticales majeures présentes dans le sang des humains et autres mammifères. M.E. Windholz, The Merck Index, Ninth Edition (1976); K. Diem and C. Lentner, Geigy Scientific Tables (1975); E. Barret-Connor et al., N.E.J.M. 315, 1519-1524 (1986). Bien que la DHEA semble servir d'intermédiaire dans la synthèse des stéroïdes des gonades, la première fonction physiologique de la DHEA n'est pas claire. Il est connu, cependant, que les concentrations plasmatiques de cette hormone, maximales dans la seconde décade de la vie, déclinent ensuite pour finalement atteindre 5% du taux originel chez les personnes âgées.

Dans certains cas, la DHEA présente des propriétés d'effecteur de l'immunité : dans R.M. Loria, T.H. Inge, S.S. Cook, A.Z. Szakl et W. Regelson (1988), J. Med. Virol., 26, 301-314, elle permet d'augmenter la survie de souris infectées par les virus humains CVB4 Edwards ou HSV2, quand elle est injectée par voie sous-cutanée en quantités de l'ordre de 1 g/kg, et ce, 4 heures avant l'infection virale. Cet effet est aussi observé lorsque la DHEA est administrée par voie orale (0,4% dans le régime) pendant 16 semaines avant l'infection.

Plus généralement, les mêmes auteurs, dans le brevet US n° 5.077.284, démontrent un effet positif sur des patients atteints du SIDA traités par des doses 400 à 2500 mg/jour, et recommandent l'utilisation de la DHEA, conjointement à un antiviral, à des doses de 25 mg/kg à 2 g/kg.

La production irréversible de DHEA à partir de la PREG est surtout connue dans le tissu corticosurrénalien et dans les gonades (E. Baulieu, Hormones, Publ. Hermann (1978)). La DHEA n'est d'ailleurs produite qu'à partir de l'adrénarche et sa quantité circulante augmente avec la puberté (E. DePeretti et E. Mappus, J. Clin. Endocr. Metab., 57, 550-556 (1983)). Dès l'âge adulte, des quantités importantes de DHEA circulent dans le sang des humains et des autres mammifères, mais ces quantités maximales dans la seconde décade de la vie humaine déclinent ensuite pour atteindre finalement moins de 10% du taux originel chez les personnes d'âge très avancé (N. Orentreich et al., J. Clin. Endocr. Metab., 59, 551-555 (1984)).

Le 5-DIOL dérive directement de la DHEA dont la fonction 17-cétone est réduite par une oxydoréductase. Ses quantités circulantes sont faibles comparées à celles de la DHEA car l'oxydoréduction agit aussi en sens inverse pour donner de la DHEA, et que des androgènes comme la testostérone peuvent en dériver même dans la peau (I. Faredin et I. Toth, Acta Med. Acad. Sci. Hung., 32, 139-152 (1975)).

Dans les gonades, la DHEA et le 5-DIOL sont successivement transformés de manière irréversible en hormones androgènes (testostérone) puis oestrogènes (oestradiol) par une série de réactions enzymatiques connues (E. Baulieu, Hormones, Publ. Hermann (1978)).

La testostérone est réduite irréversiblement dans les tissus cibles en son dérivé androgéniquement actif, la 5α-dihydrotestostérone. C'est de ce stéroïde que dérivent l'ADIOL et l'ISOA par des réactions enzymatiques réversibles (E. Baulieu, Hormones, Publ. Hermann (1978)). Les quantités circulantes de ces deux stéroïdes sont faibles chez l'adulte et sont nulles avant la puberté et presque nulles après la ménopause et l'andropause (P. Mauvais-Jarvis et G. Charransol, Nvelle. Presse Médic., 23, 1565-1569 (1973); C. Hopkinson et al., J. Steroid Biochem., 8, 1253-1257 (1977); G. Habrioux et al., Steroids, 32, 61-71 (1978)).

L'utilisation de la PREG n'a jamais été envisagée pour des applications thérapeutiques car ce stéroïde est le précurseur de l'ensemble des hormones stéroïdes. Par contre, la DHEA a des effets démontrés dans différentes conditions physiopathologiques, incluant notamment le cancer, le diabète, le vieillissement, l'obésité. Ces différents effets sont décrits dans une série d'articles collégés dans un ouvrage récent sur les effets biochimiques, biologiques et cliniques de la DHEA (The biologic role of dehydroepiandrosterone, Éditeurs: M. Kalimi et w. Regelson, Publication: Walter de Gruyter, Berlin, New York, 1990). Le mode d'action exact de la DHEA y est étudié mais non encore expliqué de manière certaine. Toutes les publications indiquent que pour obtenir un effet, la DHEA doit être administrée soit oralement, soit en sous-cutané à des doses importantes allant de 25 mg/kg à 2g/kg, et ce, en administrations répétées.

Cependant, l'utilisation de la DHEA dans une composition thérapeutique ou prophylactique se heurte à trois problèmes :
- la faible solubilité de la DHEA dans des solutions aqueuses,
- la nécessité de l'injecter plusieurs heures avant l'antigène pour obtenir l'effet recherché,
- les quantités importantes nécessaires à l'obtention d'un effet risquent d'entraîner un coût prohibitif et des effets secondaires indésirables.

De la même manière, le 5-DIOL a récemment été utilisé pour obtenir une réponse antivirale augmentée chez la souris. Les doses reconnues comme efficaces sont de 20 mg/kg à 320 mg/kg après administration sous-cutanée, ou de 0,4% du poids des aliments (R. Loria et D. Padgett, Arch. Virol., 127, 103-115 (1992)). Les effets secondaires connus du 5-DIOL limitent néanmoins considérablement son utilisation thérapeutique chez l'homme.

Enfin, le ADIOL et l'ISOA ne sont pas employés ni envisagés en tant qu'agents thérapeutiques à cause de leur action androgène relativement forte (P. Robel et al., Biochimie, 53, 81-96 (1971)).

Des stéroïdes 3β-hydroxylés comme la PREG, la DHEA, le 5-DIOL sont hydroxylés en position 7 dans de nombreux tissus comme cela a déjà été montré chez le rat (Y. Akwa et al., Biochem. J., 288, 959-964 (1992)) et la souris (R. Morfin et G. Courchay, J. Steroid Biochem. Molec. Biol., (sous presse)). Pour le ADIOL et l ' ISOA, des hydroxylations en position 6 ou 7 ont été mises en évidence chez l'homme (R. Morfin et al., Biochimie, 59, 637-644 (1977)), chez le rat (J. Guiraud, R. Morfin et al., Steroids, 34, 241-248 (1979)) et chez le chien (R. Morfin et al. Eur. J. Biochem., 109, 119-127 (1980)). Cette découverte a été confirmée et étendue chez l'homme (F. Jacolot et al., J. Steroid Biochem., 14, 663-669 (1981); S. Tunn et al., J. Steroid Biochem., 28, 257-263 (1987)) et chez le rat (J. Isaacs et al., Steroids, 33, 639-657 (1979); M. Warner et al., Endocrinology, 124, 2669-2706 (1979)). Ces hydroxylations en 6 ou 7 sont spécifiques des stéroïdes 3β-hydroxylés et le processus enzymatique opère de manière irréversible.

Il en ressort donc que les stéroïdes 6 ou 7-hydroxylés formés ne peuvent donner naissance aux hormones stéroïdes classiques telles que les minéralocorticoïdes, glucocorticoïdes, progestatifs, androgènes et oestrogènes et ils sont apparus comme inactifs dans les domaines de ces dernières hormones ( A. Sunde et al., J. Steroid Biochem., 16, 483-488 (1982); F. Celotti et al., J. Steroid Biochem., 18, 397-401 (1983)).

Divers articles récents démontrent aussi l'existence d'une association entre les systèmes endocriniens et immunitaires; il y est suggéré que la DHEA jouerait un rôle régulateur anti-glucocorticoïde (A. Meikle et al., J. Steroid Biochem. Molec. Biol., 42, 293-304 (1992)), bien que des recherches avec ce stéroïde n'aient apporté que des réponses négatives (P. Mohan et M. Cleary, Steroids, 57, 244-247 (1992)).

Le document WO 92 03925 concerne une méthode de traitement du contrôle de poids chez l'humain, par l'utilisation de certains stéroïdes substitués.

Le brevet US 4 898 694 décrit des procédés de synthèse chimique de préparation de stéroïdes dérivés de la DHEA, de l'isoandrostérone ou de l'androstane-3β,17β-diols.

Le brevet US 4 628 052 et le document DE 38 12 595 décrivent des molécules de stéroïdes correspondant à certains précurseurs des composés utiles selon la présente invention.

Le document WO 91 04030 décrit l'utilisation de stéroïdes naturels dans des compositions qui induisent la production de lymphokine par la cellule T.

Le document US 5 077 284 décrit l'utilisation de DHEA naturelle comme stimulant de la réponse immunitaire face à des agents infectieux ou immunogènes.

La présente invention a pour but de pallier les différents problèmes soulevés plus haut sur l'utilisation thérapeutique de la DHEA ou de la PREG et découle de la découverte selon laquelle, non seulement les microsomes de cerveau de rat, mais la peau, l'intestin, le colon, le coecum, la rate, le thymus et le cerveau de la souris, étaient capables de convertir la DHEA et la prégnénolone en des dérivés polaires, et plus hydrosolubles, que sont les dérivés 7α-hydroxylés. Des dérivés 7β-hydroxylés ont également été détectés. De plus, la quantité de dérivés 7α-hydroxylés augmente avec l'âge des animaux.

La présente invention est relative à l'utilisation, pour la préparation de compositions pharmaceutiques les comprenant à titre de principe actif, des composés de formule générale : 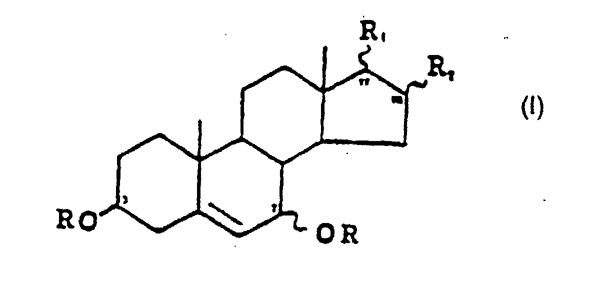 ou les formules générales (II), (III) et (IV) : 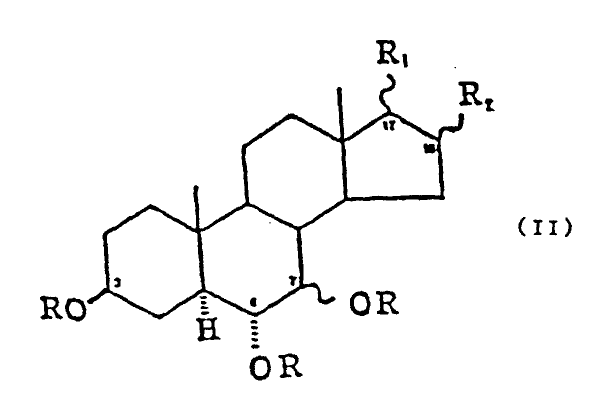 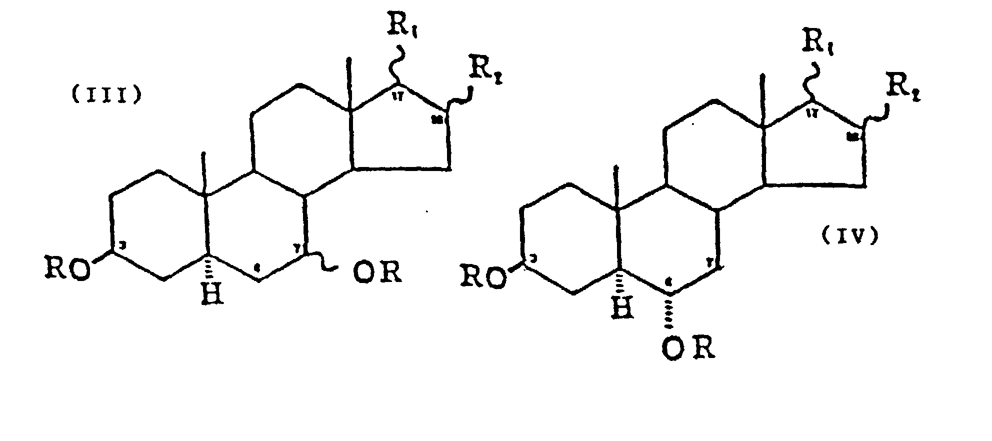 dans lesquelles les substituants R, R1 et R2 ont les significations suivantes :
- R =: ou fonction ester de 1 à 100 atomes de carbone,
- R₁: -H₂ ou =O (cétone) ,
ou β-OH (hydroxyle),
ou β-CO-R₃,
ou β-CHOH-R₃,
ou β-OH,
avec R₃ étant un groupe alcoyle comprenant de 1 à 10 atomes de carbone, de préférence méthyle, substitué ou non, R2 = -H₂ ou un halogène, ou un radical carbonitrile, les radicaux 6, 7 et en 16 peuvent être en position α ou β, et dans lesquels le cas échéant le substituant des hydrogènes en position 7 peut être une cétone,
les composés I, II, III ou IV étant associés, dans ladite composition pour le traitement par des agents anti-glucocorticoïdes, avec un véhicule pharmaceutique acceptable.

Dans les composés préférés de l'invention, R est un hydrogène, R1 est une fonction = O ou -CO-CH₃.

Dans les composés de formule I à IV les substituants des hydrogènes en positions 6 et 7 peuvent être une cétone.

En outre, dans les formules générales II à IV, les radicaux en 6 et 7 peuvent être mutuellement exclusifs (formules III et IV) ou être présents sur la même molécule (formule II).

Des composés particuliers de l'invention sont ceux pour lesquels R=H, ou R₂=H₂, ou R₁=β-CH-CH₃ ou =0, et en particulier le 7α hydroxy-iso-androstérone répondant à la formule (III).

L'invention concerne plus particulièrement les compositions pharmaceutiques dans lesquelles les composés du genre en question sont associés à un véhicule pharmaceutique approprié au mode d'administration choisi.

Les compositions pharmaceutiques de l'invention contenant à titre de principe actif un compose de formule I, II, III ou IV peuvent être utilisées dans la fabrication d'une composition pharmaceutique utilisable comme anti-glucocorticoïde, et ce, dans toutes les utilisations thérapeutiques impliquant la recherche d'une action contraire à celle des glucocorticoïdes naturels ou de synthèse, les substituants en R, R₁ et R₂, des formules I à IV pouvant en outre être simultanément R=H, R₁=cétone et R₂=H₂.

Les pathologies concernées sont toutes celles où l'utilisation d'agents glucocorticoïdes est médicalement déconseillée, ainsi que celles découlant des effets secondaires indésirables de ces mêmes glucocorticoïdes, telles que définies dans les revendications.

Parmi ces pathologies associées aux glucocorticoïdes, et aux effets secondaires indésirables dûs aux glucocorticoïdes, on peut citer, à titre d'exemples:
- le syndrome de Cushing,
- les tumeurs surrénaliennes,
- les effets diabétogènes et lipidogènes des glucocorticoïdes,
- la faiblesse, l'atrophie musculaire,
- les ulcères digestifs, l'ostéoporose, la mobilisation des acides gras et l'accumulation des graisses résultant de la prise de glucocorticoïdes,
- la cataracte chez l'adulte et la diminution de la croissance des enfants traités par les glucocorticoïdes,
- les dysménorrhées, les insomnies et les états dépressifs résultant de traitements ou d'actions glucocorticoïdes excessifs.

L'effet pharmacologique maximal de ces composés est obtenu par toute voie d'administration amenant la molécule active au niveau des cellules visées, ce qui comprend notamment l'administration per os, sous-cutanée, per-cutanée, intramusculaire ou topique.

Un dosage avantageux des composés de formule I ou II utilisés dans la fabrication de la composition thérapeutique est de 1 à 100 mg/kg chez les mammifères.

Aux fins d'administration d'un stéroïde, des solvants organiques sont habituellement utilisés dans la pharmacopée animale et humaine. Il s'agit notamment:
- du DMSO (diméthyl sulfoxyde) pour l'injection intramusculaire ou sous-cutanée;
- les solvants pour les stéroïdes injectables par voie sous-cutanée ou intramusculaire qui sont couramment des huiles végétales (ricin, olive, arachide,...) quelques fois hydrogénées ou neutralisées. Quelques stéroïdes sont aussi injectés sous forme de suspension dans la carboxyméthyl cellulose;
- les administrations transcutanées (pommades, gels) utilisant surtout des mélanges (galéniques et excipients) où l'on retrouve des alcools (éthylène, propylène glycols), des alcools gras (stéarylique, octyl-2-dodécanol, en C₂₀ ...), de la vaseline, des huiles végétales (ricin, olive...).
   De nouvelles formulations permettent aussi une absorption transcutanée ou transépithéliale retardée. Il s'agit d'inclusions dans le polyvinylpyrrolidone ou dans le polyéthylène glycol ou dans des matrices polymérisées avec de la silice fumée.
   L'administration au niveau du colon peut utiliser la voie rectale avec des suppositoires (beurre de cacao) mais aussi la voie orale avec les cyclodextrines qui emprisonnent le principe actif hydrophobe et le libèrent une fois détruit dans le colon (J. Szetli, Pharmaceut. Technol. Int., 3, 16-24 (1991)).
   L'invention concerne enfin les médicaments utilisables comme effecteurs de l'immunité ou destinés à la prévention ou au traitement de maladies ou de symptomes résultant de l'action de glucocorticoïdes caractérisés en ce qu'ils contiennent un composé de formule I ou de formule II en association avec un véhicule pharmaceutique approprié au mode d'administration choisi.
   Les différents exemples qui suivent montrent sans caractère limitatif les effets thérapeutiques des dérivés 7α ou 7β-hydroxylés, aussi bien comme effecteurs de l'immunité que comme agents anti-glucocorticoïdes.

### I- EFFET EFFECTEUR DE L'IMMUNITE [seulement par illustration]

L'effet effecteur de l'immunité a été éprouvé par injection sous-cutanée concomitante à celle d'un antigène et du dérivé 7α ou 7β-hydroxylé à des souris C57BL/6, selon un protocole d'immunisation classique. (Methods in Enzymology (1980), vol. 70).

L'effet du composé comme effecteur de l'immunité cellulaire est mesuré par la quantité d'IgG circulantes dirigées contre l'antigène, et ce, 21 jours après l'injection, après une injection de rappel à 14 jours.

Les exemples suivants, sans caractère limitatif, décrivent l'invention.

### - EXEMPLE I : Effet de la 7αOH-DHEA et de la 7βOH-DHEA:

### ·Préparation (synthèse) des 7α- et 7β-hydroxy-déhydroépiandrostérone :

L'acétate de DHEA (1) (1 g, Sigma D-4500), est transformé en 3β-acétoxy, 7α-bromoandrost-5-ène-17-one (2) par le procédé publié par J. ORR and J. BROUGHTON (J. Org. Chem. (1970) 35, 1126-1129) utilisant le N-bromosuccinimide sous irradiation lumineuse.

Le dérivé bromé obtenu est transformé en un mélange de 3β, 7α(β)-diacétoxy-androst-5-ène-17-one (3) en traitant par le mélange acétate de K/acide acétique selon A. NUSSBAUM et al. (J. Am. Chem. Soc. (1960) 82, 2641).

Le mélange obtenu est saponifié dans CO₃K₂ méthanolique à reflux pendant 3 heures. Après extraction des stéroïdes saponifiés, le 3β, 7α-dihydroxy-androst-5-ène-17-one (4) est séparé du 3β, 7β-dihydroxy-androst-5-ène-17-one (5) par chromatographie préparative sur gel de silice élué par l'acétate d'éthyle. L'épimère 7β est élué en premier complètement séparé du stéroïde 7α qui est élué ensuite.

Le (4) est cristallisé dans acétate d'éthyle/n-hexane. Les cristaux (120 mg) ont un point de fusion de 182-183°C correspondant à ceux trouvés par R. DODSON et al. (J. Am. Chem. Soc. (1959) 81, 6295-6297) et par G. DEFAYE et al. (J. Steroid Biochem. (1978) 9, 331-336).

Le (5) est cristallisé de la même manière (280 mg). Les cristaux ont un point de fusion de 214-215°C correspondant à ceux trouvés par les mêmes auteurs que ci-dessus.

Le passage de (4) et (5) en chromatographie en phase gazeuse couplée à la spectrométrie de masse sous la forme de dérivés di-triméthylsilyloxy donne pour chacun un ion moléculaire à 448 correspondant à la masse attendue et les mêmes temps de rétention et profils de fragmentation que celui des mêmes dérivés de (4) et (5) de référence (fournis par H.A. LARDY, Université du Wisconsin).

### . Protocole d'immunisation :

Trente cinq lots de 5 souris C57BL/6 âgées de 6 semaines subissent une injection sous-cutanée de 200 µg de lysozyme de blanc d'oeuf de poule le premier et le quatorzième jour d'un protocole d'immunisation. Des solutions de DHEA (Sigma D.4500) dans le diméthylsulfoxyde (DMSO) sont injectées de la même manière à des temps variables avant la première injection de lysozyme. Des quantités comprises entre 0,25 et 2 g/kg sont utilisées. Dix autres lots de 5 souris subissent une injection sous-cutanée de 6,25 à 50 mg/kg de 7α-hydroxy-DHEA ou de 7β-hydroxy-DHEA en solution dans le DMSO au même moment que la première du lysozyme. Deux lots ne reçoivent que le DMSO avec le lysozyme (contrôles). Les blancs correspondant à un lot de 5 souris ne subissant aucune injection.

Trois semaines après les premières injections, les animaux alors âgés de 9 semaines sont décapités. Le sang de chaque animal est recueilli, et la présence d'immunoglobulines G (IgG) anti-lysozyme est mesurée dans des dilutions en série des sérums par un dosage immunoenzymatique utilisant des IgG de chèvre anti-IgG de souris (Fc) marquées à la peroxydase.

Les résultats sont indiqués dans les lignes 1, 3, 6 et 7 du tableau 1, où l'effet particulièrement marqué des faibles doses de la 7αOH-DHEA apparaît. La 7βOH-DHEA a un effet équivalent à celui de la DHEA injectée 1 heure avant l'antigène, mais à des doses 20 fois plus faibles.

### - EXEMPLE II : Effet du 7α OH et 7β OH prégnénolone :

### ·Préparation (synthèse) des 7α- et 7β-hydroxy-prégnénolone :

L'acétate de prégnénolone (1) (Sigma, P-9254), est transformé en 3β-acétoxy-7α-bromoprégn-5-ène-20-one (2) par le procédé publié par J. ORR and J. BROUGHTON (J. Org. Chem. (1970), 35, 1126-1129) utilisant le N-bromosuccinimide sous irradiation lumineuse.

Le dérivé bromé obtenu est transformé en un mélange de 3β, 7α(β)-diacétoxy-prégn-5-ène-20-one (3) en traitant par le mélange acétate de K/acide acétique selon A. NUSSBAUM et al. (J. Am. Chem. Soc. (1960) 82, 2641).

Le mélange obtenu est saponifié dans CO₃K₂ méthanolique à reflux pendant 3 heures. Après extraction des stéroïdes saponifiés, le 3β, 7α-dihydroxy-prégn-5-ène-20-one (4) est séparé du 3β, 7β-dihydroxy-prégn-5-ène-20-one (5) par chromatographie préparative sur gel de silice élué par l'acétate d'éthyle. L'épimère 7β est élué en premier complètement séparé du stéroïde 7α qui est élué ensuite.

Le (4) est cristallisé dans l'acétate d'éthyle/n-hexane. Les cristaux sont contrôlés par résonance magnétique nucléaire (200 MHz) , 0.55 (3H, s, Me-18) , 0.99 (3H, s, Me-19), 2.13 (3H, s, Me-21), 3.62 (1H, m, H-3) , 3.86 (1H, s, H-7), 5.62 (1H, d, H-6). Le passage du dérivé di-triméthylsilyloxy de (4) en chromatographie en phase gazeuse couplée à la spectrométrie de masse donne l'ion moléculaire attendu à 476 et le profil de fragmentation correspondant.

Le (5) est cristallisé dans l'acétate d'éthyle/n-hexane. Les cristaux sont contrôlés par résonance magnétique nucléaire (200 MHz), 0.63 (3H, s, Me-18), 1.05 (3H, s, Me-19), 2.12 (3H, s, Me-21), 3.53 (1H, m, H-3) , 3.83 (1H, d, H-7), 5.30 (1H, s, H-6). Le passage du dérivé di-triméthylsilyloxy de (5) en chromatographie en phase gazeuse couplée à la spectrométrie de masse donne l'ion moléculaire attendu à 476 et le profil de fragmentation correspondant.

### · Protocole d'immunisation :

Celui-ci est réalisé de la même façon que dans l'exemple I.

Les résultats sont indiqués lignes 2 et 5 du tableau 1.

### - EXEMPLE III : Effet des androst-5-ène-3β, 7α(β), 17β-triol (7αOH ADIOL et 7βOH ADIOL) :

### . Préparation (synthèse) des androst-5-ène-3β, 7α(β), 17β-triol (7αOH ADIOL et 7βOH ADIOL) :

L'acétate de déhydroépiandrostérone (DHEA) (1) (Sigma, D-4500), est oxydé en position 7 par le complexe CrO₃-diméthylpyrazole selon SALMOND et al. (J. Org. Chem. (1978) 43, 2057) et conduit au 3β-acetoxy-androst-5-ène-7, 17-dione (2). Ce produit est identique (point de fusion, Rf chromatographique, absorption UV, spectre de masse) à celui fourni en référence par J. JACQUES (Collège de France).

(2) est réduit par NaBH₄ dans le méthanol et conduit au mélange des épimères 3β-acétoxy- 7α(β), 17β-dihydroxy-androst-5-ène (3). Après saponification du mélange (3) dans le KOH/methanol, les androst-5-ène-3β , 7α, 17β-triol (4) et androst-5-ène-3β, 7β, 17β-triol (5) sont successivement obtenus et complètement séparés par chromatographie sur gel de silice élué par l'acétate d'éthyle.

(4) et (5) sont cristallisés dans l'acétone/n-hexane. Les cristaux de (4) ont un point de fusion de 272-273°C correspondant à celui rapporté par G. DEFAYE et al. (J. Steroid Biochem. (1978) 9, 331-336) et son dérivé tri-triméthyl-silyloxy présente, après chromatographie en phase gazeuse couplée à la spectrométrie de masse l'ion moléculaire attendu à 522 et le profil de fragmentation correspondant.

Les cristaux de (5) ont un point de fusion de 244-245°C correspondant à celui rapporté par G. DEFAYE et al. J. Steroid Biochem. (1978) 9, 331-336), et son dérivé tri-triméthylsilyloxy présente, après chromatographie en phase gazeuse couplée à la spectrométrie de masse l'ion moléculaire attendu à 522 et le profil de fragmentation correspondant.

### · Immunisation :

Un lot de 5 souris subit l'injection sous-cutanée de 125 mg/kg de 5-androstène-3β, 17β-diol (ADIOL) une heure avant la première injection de lysozyme, et deux lots de 5 souris sont traités par 25 mg/kg de 7α-hydroxy-ADIOL ou de 7β-hydroxy-ADIOL au moment de la première injection de lysozyme.

### ·Résultats :

Les résultats sont indiqués lignes 4, 8 et 9 du tableau 1 ci-dessous.

Tableau 1 : Mesures immunoenzymatiques des IgG anti-lysozyme présentes dans le sérum de souris C57BL/6 immunisées contre le lysozyme après traitement par la prégnénolone (PREG) ou la DHEA ou l'androst-5-ène-3β 17β-diol ou leurs dérivés 7-hydroxylés ou par aucun stéroïde.

La DHEA ou la PREG ou l'ADIOL est injecté sous la peau 1 heure avant la première injection de lysozyme. Les dérivés 7-hydroxylés sont administrés au moment de l'injection du lysozyme. La mesure immunoenzymatique des IgG anti-lysozyme dans des dilutions sériées des sérums de chaque animal utilise des IgG de chèvre anti-IgG de souris marquées à la peroxydase sur des plaques 96-puits traitées au lysozyme. La moyenne des densités optiques mesurées dans chaque lot de 5 souris est calculée ainsi que la déviation standard (SD).

**TABLEAU 1**

| Stéroïde injecté (dose) | | Densités optiques ± SD dans les dilutions sériques | | |
|---|---|---|---|---|
| 1/180 | 1/540 | | | |
| 1. | Contrôles (nulle) | 0,15±0,01 | 0,13±0,01 | 0,10±0,01 |
| 2. | PREG (0,5 g/kg) | *4,67±1,21 | 1,83±0,48 | 0,56±0,15 |
| 3. | DHEA (1,0 g/kg) | 0,40±0,06 | 0,26±0,04 | 0,14±0,02 |
| 4. | ADIOL (125 mg/kg) | 0,26±0,09 | 0,37±0,06 | 0,22±0,03 |
| 5. | 7 -hydroxy-PREG (6,25 mg/kg) | *4,46±0,30 | *2.20±0,14 | 0,77±0,05 |
| 6. | 7 -hydroxy-DHEA (6,25 mg/kg) | 1,21±0,37 | 0,74±0,21 | 0,43±0,12 |
| 7. | 7β-hydroxy-DHEA (50,0 mg/kg) | 0,46±0,06 | 0,31±0,04 | 0,13±0,01 |
| 8. | 7 -hydroxy-ADIOL (25,0 mg/kg) | 0,15±0,02 | 0,14±0,01 | 0,11±0,01 |
| 9. | 7β-hydroxy-ADIOL (25,0 mg/kg) | 0,18±0,06 | 0,18±0,02 | 0,12±0,01 |

| | | | | |
|---|---|---|---|---|
| * Calculé à partir des mesures sur des dilutions supérieures du sérum. | | | | |

La PREG, la DHEA et l'ADIOL augmentent les concentrations sériques d'IgG anti-lysozyme lorsque ces stéroïdes sont administrés au moins une heure avant l'injection du lysozyme. Les meilleurs réponses sont obtenues avec 0,5 g/kg de PREG et 1 g/kg de DHEA (tableau 1). Lorsque ces stéroïdes sont injectés au même moment que le lysozyme, les quantités d'IgG restent identiques à celles trouvées chez les contrôles.

Par contre, les dérivés 7α-hydroxylés de la PREG et de la DHEA déclenchent une production accrue d ' IgG lorsqu'ils sont administrés au même moment que le lysozyme. La quantité la plus faible injectée (6,25 mg/kg) est la plus active (tableau 1). L'injection dans ces conditions de 50 mg/kg de 7β-hydroxy-DHEA donne une réponse identique à celle de 1g/kg de DHEA administrée 1 heure avant celle du lysozyme (tableau 1). Les dérivés 7-hydroxylés de l'ADIOL ne déterminent, à la dose testée, qu'une très faible augmentation des IgG anti-lysozyme sériques (tableau 1).

Ces résultats relient l'hydroxylation tissulaire des précurseurs naturels des hormones stéroïdes au déclenchement de la réponse immunitaire. Le temps de latence pour l'action de la PREG ou de la DHEA sur l'augmentation des IgG anti-lysozyme implique la transformation de ces stéroïdes en métabolites actifs. La démonstration de leur hydroxylation en 7α dans les tissus cutanés, et celle de l'effet immédiat de ces dérivés 7α-hydroxylés sur la production accrue d'IgG anti-lysozyme contribuent à le prouver. L'étude des hydroxylations de stéroïdes 3β-hydroxylés dans divers tissus chez plusieurs espèces (R. Morfin, S. Di Stéfano, J.F. Charles and H.H. Floch (1977) Biochimie, 59, 637-644; J.T. Isaacs, I.R. Mc Dermott and D.S. Coffey (1979), Steroids, 33, 675-692; M. Warmer, P. Tollet, M. Strömstedt, K. Carlström and J.A. Gustafsson (1989), J. Endocrinol., 122, 341-349 ; Y. Akwa, R. Morfin, P. Robel and E.E. Baulieu, (1992), Biochem. J., 288, 959-964 ; R. Morfin, résultats non publiés sur la souris) a prouvé que l'hydroxylation en 7 est majoritaire en 7α mais avec de faibles quantités en 7β. Il apparaît aussi que la 7α-hydroxy-DHEA est bien plus active que son épimère en 7β . Il n'en reste pas moins que les épimères en 7β et les compositions les contenant doivent également être considérés comme faisant partie de l'invention.

Ces exemples sont donnés à titre d'illustration mais ne sont pas limitatifs
- ni de la dose utilisée,
- ni de la présence d'une double liaison 5-6 sur le stéroïde,
- ni de l'effet observé sur la synthèse des IgG.

La DHEA 7α-hydroxylée constitue un métabolite naturel de la DHEA, ce qui pourrait expliquer les difficultés antérieurement observées avec la DHEA. En effet, les quantités de DHEA nécessaires à la mise en évidence des effets physiologiques observés par divers auteurs (The biologic role of Dehydroepiandrostérone, Editeurs M. Kalimi, W. Regelson, Publication Walter de Gruyter, Berlin, New York, 1990), et le fait qu'un effet stimulateur de l'immunité nécessitait une injection préalable à celle de l'antigène, s'expliquent-ils peut-être à posteriori par le fait que les activités observées pour la DHEA devaient passer par une nécessaire métabolisation préalable de ce stéroïde.

### II- EFFET ANTIGLUCOCORTICOIDE :

L'effet anti-glucocorticoïde de ces molécules a été prouvé par les études de leur compétition avec la Dexaméthazone radio-marquée pour sa rétention nucléaire dans les cellules de tissus hépatique, cérébral, splénique et thymique chez la souris C57BL/6, selon le protocole publié par P. Mohan et M. Cleary sur des hépatocytes de rat (Steroids, 57, 244-247 (1992)). Ce protocole expérimental est lui-même une adaptation de la méthode de D. Colvar et al; (Clin. Chem., 34, 363-369 (1988)).

Les exemples suivants, sans caractère limitatif décrivent l'invention.

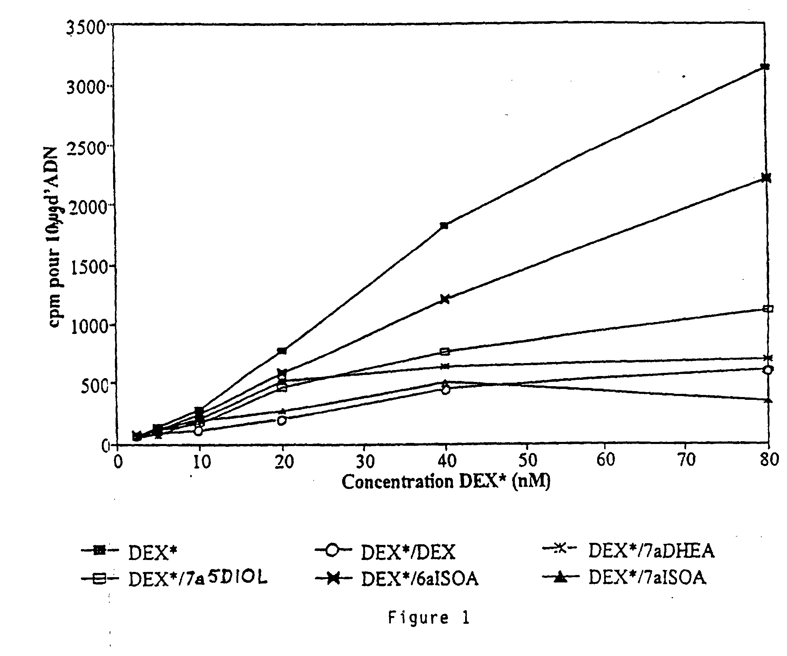

La figure 1 représente l'effet anti-glucocorticoïde de différents dérivés testés, mesurés par compétition avec la dexamethasone radioactive quant à leur liaison sur les noyaux d'hépatocytes de rat.

Les hépatocytes isolés sont incubés pendant 1 heure à température ambiante avec des concentrations croissantes (2-80 nM) de ³H-Dexaméthazone (DEX*). Les mêmes incubations sont répétées en présence d'un excès de 100 fois (200-8000 nM) de Dexaméthazone non radioactive (DEX*/DEX) ou de 7α-hydroxy DHEA (DEX*/7aDHEA) ou de 7α-hydroxy-5DIOL (DEX*/7a5DIOL) ou de 6α-hydroxy-ISOA (DEX*/6aISOA) ou de 7α-hydroxy-ISOA (DEX*/7aISOA). Les noyaux des hépatocytes sont ensuite isolés et purifiés et la radioactité correspondant à la DEX* retenue est mesurée et exprimée en cpm/10µg d'ADN. L'aire sous la courbe DEX* correspond à un déplacement nul de la radioactivité (zéro). L'aire sous la courbe DEX*/DEX qui correspond au déplacement de la radioactivité par la même molécule est pris arbitrairement comme 100. Les aires sous les courbes obtenues avec les autres stéroïdes permettent de calculer par interpolation entre les aires zéro et 100 les coefficients de déplacement de la liaison nucléaire de la DEX*.

Le fait que les stéroïdes testés qui se sont révélés les plus actifs soient des dérivés naturels des hormones stéroïdes androgènes prend pleinement sa signification au travers des nombreuses interactions observées au cours de traitements par des associations de glucocorticoïdes et de stéroïdes androgènes anabolisants (O. Linet, Progr. Drug Res., 14, 139-195 (1970)). Les effets "anti-glucocorticoïdes" observés à la suite de ces traitements sont très variables et peuvent être maintenant expliqués par la nécessité d'une métabolisation préalable des stéroïdes androgènes administrés afin que des dérivés 7-hydroxylés actifs soient produits.

Cette constatation amène donc à considérer des molécules stéroïdes de synthèse reconnues comme possédant un effet semblable à celui de leur parent naturel, telle la 16-Bromo-ISOA (A. Schwartz et al., Adv. Cancer Res., Publ. Academic Press, 51, 391-424 (1988); G. Gordon et al., Adv. Enzyme Regul., Publ. Pergamon Press, 26, 355-382 (1987)). La transformation possible d'un tel stéroïde en un dérivé 6- ou 7-oxygéné particulièrement actif nous amène donc à considérer que ces dérivés et les compositions les contenant font partie de l'invention.

Le mécanisme exact de l'interaction des stéroïdes 6- et 7-oxygénés avec le complexe Glucocorticoïde-récepteur-ADN n'est pas résolu ; néanmoins, l'effet anti-glucocorticoïde observé dans chacun des exemples donnés peut être considéré comme une illustration non limitative des dérivés utilisables dans la mesure où il répond à l'une des formules I ou II ci-dessus. Ces exemples ne sont pas non plus limitatifs de la dose, ni du mode d'administration utilisés, ni des effets thérapeutiques résultant de leur utilisation.

### - EXEMPLE IV: Effet anti-glucocorticoïde de la 7α-hydroxy-DHEA et de la 7β-hydroxy-DHEA:

### Préparation (synthèse) des 7α- et 7β-hydroxy-DHEA:

Celle-ci est effectuée comme dans l'exemple I.

### Test d'activité anti-glucocorticoïde:

Des souris C57BL/6 âgées de 7 à 12 semaines sont traitées par la Métyrapone (25mg/kg) 24 heures et 12 heures avant le sacrifice des animaux, de manière à diminuer leur taux de glucocorticoïdes naturels et à augmenter les concentrations cellulaires en récepteurs spécifiques des glucocorticoïdes (R. Rupprecht et al., J. Neuroendocr., 2, 803-806 (1990)). Les souris sont anesthésiées à l'éther et saignées, puis perfusées par la voie intracardiaque avec du sérum physiologique contenant de la collagénase B (0.03g/100ml) pendant 10 minutes. Le foie, le cerveau, la rate et le thymus sont prélevés et les cellules de chaque organe sont récupérées par la méthode décrite (P. Mohan et M. Cleary, Steroids, 57, 244-247 (1992)). Les quantités de cellules obtenues sont estimées sur la base de la mesure de l'ADN contenu dans les préparations. Les cellules sont incubées pendant 1 heure à température ambiante en présence de quantités croissantes (2nM à 80nM) de Dexaméthazone tritiée additionnée ou non d'une quantité 100 fois supérieure de Dexaméthazone non radioactive ou d'un des composés à tester.

Les noyaux sont récupérés et leur contenu radioactif mesuré comme décrit par le auteurs cités plus haut.

Les résultats correpondants sont portés sur une courbe (Figure 1) où la radioactivité mesurée par 10µg d'ADN cellulaire est exprimée en fonction de la molarité de Dexaméthazone tritiée incubée. Le déplacement attendu de la Dexaméthazone tritiée par la Dexaméthazone non radioactive est observé. L'aire sous la courbe de la Dexaméthazone tritiée seule est mesurée et prise comme zéro. L'aire sous la courbe de la Dexaméthazone tritiée additionnée d'un excès de 100 fois en Dexaméthazone non radioactive est mesurée et prise comme 100. Les aires sous courbe, pour chacun des stéroïdes non radioactifs testés par addition individuelle d'une quantité 100 fois supérieure à celle de la Dexaméthazone tritiée, sont mesurées et leur coefficient de déplacement de la Dexaméthazone tritiée liée dans les noyaux calculé par interpolation sur la base du zéro et du 100.

Les résultats obtenus pour chacun des quatre organes avec la 7α-hydroxy-DHEA et la 7β-hydroxy-DHEA sont présentés dans le tableau 2 aux lignes 1 et 2. Le déplacement de la Dexaméthazone tritiée qu'ils occasionnent dans les noyaux prouve leur action anti-glucocorticoïde, la 7α-hydroxy-DHEA étant plus efficace que l'épimère 7β.

### - EXEMPLE V: Effet anti-glucocorticoïde de la 7α-hydroxy-PREG et de la 7β-hydroxy-PREG:

### Préparation (synthèse) des 7α- et 7β-hydroxy-PREG:

Cette préparation est identique à celle décrite dans l'exemple II.

### Test d'activité anti-glucocorticoïde:

Celui-ci est réalisé de la même façon que dans l'exemple IV.

Les résultats sont indiqués aux lignes 3 et 4 du tableau 2. Le déplacement de la Dexaméthazone tritiée qu'ils occasionnent dans les noyaux prouve leur effet anti-glucocorticoïde.

### - EXEMPLE VI: Effet des 5-androstène-3β,7α(β),17β-triols (7α-hydroxy-5DIOL et 7β-hydroxy-5DIOL):

### Préparation (synthèse) des 7α-hydroxy-5DIOL et 7β-hydroxy-5DIOL:

La préparation est identique à celle décrite dans l'exemple III.

### Test d'activité anti-glucocorticoïde:

Celui-ci est réalisé de la même manière que dans l'exemple IV.

Seul le 7α-hydroxy-5DIOL est testé sur les noyaux d'hépatocytes. Les résultats sont indiqués à la ligne 5 du tableau 2. Le déplacement de la Dexaméthazone tritiée liée dans les noyaux d'hépatocytes par le 7α-hydroxy-5DIOL prouve son effet anti-glucocorticoïde.

### - EXEMPLE VII: Effet des dérivés 6- et 7-oxygénés de stéroïdes 3β-hydroxylés et 5α-réduits:

### Préparation (synthèse) des 3β-hydroxy-5α-androstane-7,17-dione (7-oxo-ISOA), 3β , 6α-dihydroxy-5α-androstane-17-one 6α-hydroxy-ISOA), 3β, 7α-dihydroxy-5α-androstane-17-one (7α-hydroxy-ISOA), 3β , 7β-dihydroxy-5α-androstane-17-one (7β-hydroxy-ISOA), 5α-androstane-3β, 7α, 17β-triol (7α-hydroxy-ADIOL) et 5α-androstane-3β, 7β,17β-triol (7β-hydroxy-ADIOL):

La synthèse, l'identification et les propriétés chromatographiques de ces stéroïdes ont été effectuées par nos soins et publiées en détail (A. Kerebel, R. Morfin, F. Berthou et al., J. Chromatogr., 140, 229-244 (1977)).

Deux autres stéroïdes sont commerciaux: la 3β-hydroxy-5-pregnène-7,20-dione (7-oxo-PREG) (Steraloids Inc., Q5400), et la 3β-hydroxy-5-androstène-7,17-dione (7-oxo-DHEA) (Steraloids Inc., A8320).

### Tests d'activité anti-glucocorticoïde:

Ceux-ci sont réalisés avec ces molécules de la même façon que dans l'exemple I. Les résultats obtenus sont indiqués aux lignes 6,7,8,9,10,11 et 12 du tableau 2 où les dérivés de l'ISOA et de l'ADIOL se révèlent tous très actifs.

### Légende du Tableau 2 :

Les stéroïdes suivants, désignés de 1 à 13 sont: 1. 7α-hydroxy-DHEA (7α-OH-DHEA), 2. 7β-hydroxy-DHEA (7β-OH-DHEA), 3. 7α-hydroxy-PREG (7β-OH-PREG), 4. 7β-hydroxy-PREG (7β-OH-PREG), 5. 7α-hydroxy-5DIOL (7α-OH-5DIOL) , 6. 7α-hydroxy-ISOA (7α-OH-ISOA), 7. 7β-hydroxy-ISOA (7β-OH-ISOA), 8. 7α-hydroxy-ADIOL (7α-OH-ADIOL), 9. 6α-hydroxy-ISOA (6α-OH-ADIOL), 10. 7-oxo-PREG, 11. 7-oxo-DHEA, 12. 7-oxo-ISOA, 13. Dexaméthazone (DEX).

Le déplacement de la liaison nucléaire de la Dexaméthazone tritiée par une concentration 100 fois supérieure de chacun de ces stéroïdes est mesuré comme indiqué dans la Figure 1. L'absence de stéroïde compétiteur est prise comme zéro, et la présence de Dexaméthazone non radioactive prise comme 100. les coefficients de déplacement entrainés par chacun des autres stéroïdes sont calculés sur cette base, chaque résultat étant la moyenne de triplicata.

**TABLEAU 2**

| Stéroïde testé | | Foie | Cerveau | Thymus | Rate |
|---|---|---|---|---|---|
| 1. | 7α-OH-DHEA | 95 | 49 | 85 | 91 |
| 2. | 7β-OH-DHEA | 80 | 65 | - | - |
| 3. | 7α-OH-PREG | 34 | 38 | 94 | 81 |
| 4. | 7β-OH-PREG | 82 | - | - | - |
| 5. | 7α-OH-5DIOL | 88 | - | - | - |
| 6. | 7α-OH-ISOA | 102 | - | - | - |
| 7. | 7β-OH-ISOA | 68 | - | - | - |
| 8. | 7α-OH-ADIOL | 83 | 96 | 96 | 125 |
| 9. | 6α-OH-ISOA | 63 | - | - | - |
| 10 | 7-oxo-PREG | 35 | - | - | - |
| 11 | 7-oxo-DHEA | 28 | - | - | - |
| 12 | 7-oxo-ISOA | 84 | - | - | - |
| 13 | DEX | 100 | 100 | 100 | 100 |

Les résultats présentés ci-dessus montrent que les dérivés 7-hydroxylés de la DHEA, de l'ISOA et du 5-DIOL déplacent activement la Dexaméthazone tritiée de sa liaison nucléaire. Les déplacements avec les autres stéroïdes sont significatifs mais moins marqués. Le fait que ces résultats soient exprimés par rapport au déplacement obtenu par des quantités équivalentes de Dexaméthazone non radioactive, et notre observation précédente (demande de brevet français n° 92.12548) démontrant un effet immunoactivateur de ces mêmes stéroïdes chez la souris qui implique le blocage de l'effet immunosuppresseur connu des glucocorticoïdes, conduisent à proposer l'utilisation de stéroïdes testés dans une composition pharmaceutique pour un traitement anti-glucocorticoïde.

La Dexaméthazone est un stéroïde de synthèse qui est-peu métabolisé (H. Siebe et al., Renal Physiol. Biochem., 16, 79-88 (1993)) et qui possède une affinité pour le récepteur cytosolique des glucocorticoïdes supérieure à celle des glucocorticoïdes naturels tels que la corticostérone ou le cortisol. Le fait que la liaison du complexe Dexaméthazone-récepteur cytosolique sur les sites de reconnaissance nucléaires soit diminuée par les stéroïdes testés confirme la potentialité d'un effet anti-glucocorticoïde. Par ailleurs, nos travaux (non rapportés ici) indiquent qu'il n'existe pas de récepteurs cytosoliques ou nucléaires spécifiques de la 7α-hydroxy-DHEA, mais son action conduisant à l'activation des processus immunitaires peut être considérée comme essentiellement anti-glucocorticoïde.

## Revendications

1. utilisation d'un composé de formule I, II, III, ou IV, ci-dessous 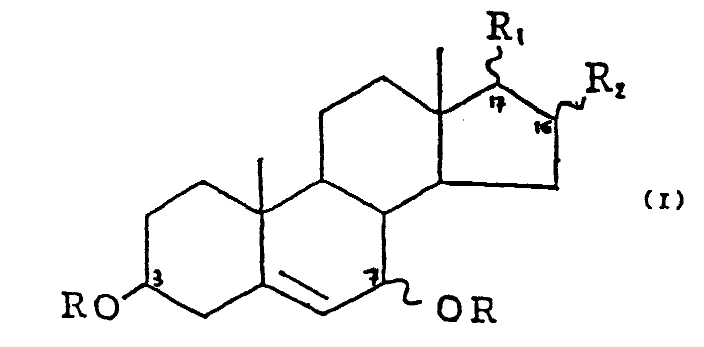 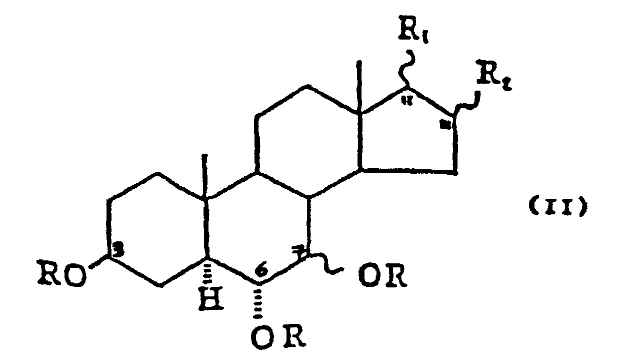 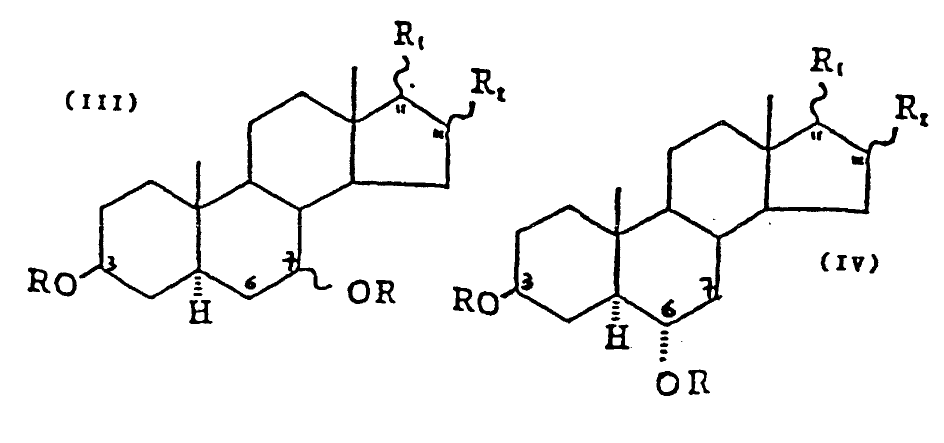 et dans lesquelles :
R = -H ou est une fonction ester de 1 à 100 atomes de carbone,
R₁ = -H₂ ou =O (cétone),
ou β-CO-R₃,
ou β-CHOH-R₃,
ou β-OH,
R₃ est un groupe alcoyle comprenant de 1 à 10 atomes de carbone, de préférence méthyle, substitué ou non,
R₂ = -H₂ ou un halogène, ou un radical carbonitrile,
les radicaux en 6, 7 et en 16 pouvant être en position a ou β, et dans lesquels le cas échéant le substituant des hydrogènes en position 7 peut être une cétone, dans la fabrication d'une composition pharmaceutique pour le traitement des pathologies associées aux glucocorticoïdes et des effets secondaires indésirables dus aux glucocorticoïdes, ces pathologies et effets secondaires étant sélectionnés dans la liste constituée :
- du syndrome de Cushing,
- des tumeurs surrénaliennes,
- des effets diabétogènes et lipidogènes des glucocorticoïdes,
- de l'atrophie musculaire et de la faiblesse,
- des ulcères digestifs,
- de l'ostéoporose,
- de la fragilité osseuse,
- de la cataracte,
- de la diminution de croissance des enfants,
- des insomnies,
- des états dépressifs,
- des dysménorrhées,
les composés I, II, III et IV étant associés dans ladite composition avec un véhicule pharmaceutique acceptable.

2. Utilisation selon la revendication 1, **caractérisée en ce que** R = H.

3. Utilisation selon la revendication 1 ou 2, **caractérisée en ce que** R₂ = H₂.

4. Utilisation selon la revendication 1, **caractérisée en ce que** R₁ =β-CO-CH₃.

5. Utilisation selon la revendication 1, **caractérisée en ce que** le composé III est le 7 α-hydroxy-iso-androstérone.

6. Utilisation d'un composé de formule I, II, III ou IV tel que défini à l'une au moins des revendications 1 à 5, pour la préparation d'une composition pharmaceutique pour le traitement des manifestations pathologiques du syndrome de Cushing ou des tumeurs surrénaliennes.

7. Utilisation d'un composé de formule I, II, III ou IV tel que défini à l'une au moins des revendications 1 à 5, pour la préparation d'une composition pharmaceutique pour la prévention et le traitement des effets diabétogènes et lipidogènes dues aux glucocorticoïdes.

8. Utilisation d'un composé de formule I, II, III ou IV tel que défini à l'une au moins des revendications 1 à 5, pour la préparation d'une composition pharmaceutique pour la prévention et le traitement de l'atrophie musculaire et de la faiblesse causés par les glucocorticoïdes.

9. Utilisation d'un composé de formule I, II, III ou IV tel que défini à l'une au moins des revendications 1 à 5, pour la préparation d'une composition pharmaceutique pour la prévention et le traitement des ulcères digestifs causés par l'utilisation ou un excès de glucocorticoïdes.

10. Utilisation d'un composé de formule I, II, III ou IV tel que défini à l'une au moins des revendications 1 à 5, pour la préparation d'une composition pharmaceutique pour la prévention et le traitement de l'ostéoporose et de la fragilité osseuse résultant de l'action des glucocorticoïdes naturels ou de synthèse.

11. Utilisation d'un composé de formule I, II, III ou IV tel que défini à l'une au moins des revendications 1 à 5, pour la préparation d'une composition pharmaceutique pour la prévention et le traitement de la cataracte résultant de l'action des glucocorticoïdes naturels ou de synthèse.

12. Utilisation d'un composé de formule I, II, III ou IV tel que défini à l'une au moins des revendications 1 à 5, pour la préparation d'un composition pharmaceutique pour la prévention de la diminution de la croissance des enfants traités par les glucocorticoïdes.

13. Utilisation d'un composé de formule I, II, III ou IV tel que défini à l'une au moins des revendications 1 à 5, pour la préparation d'une composition pharmaceutique pour la prévention et le traitement des insomnies et des états dépressifs résultant de l'action des glucocorticoïdes naturels ou de synthèse.

14. Utilisation d'un composé de formule I, II, III ou IV tel que défini à l'une au moins des revendications 1 à 5, pour la préparation d'une composition pharmaceutique pour la prévention des dysménorrhées résultant de l'action des glucocorticoïdes.

15. Utilisation selon les revendications 1 à 14, **caractérisée en ce que** le composé et le véhicule pharmaceutique sont associés sous une forme galénique permettant une administration soit per os, soit per cutanée, soit topique.

16. Utilisation selon les revendications 1 à 15, **caractérisée par le fait que** le composé est dosé de façon à permettre une administration de 1 à 100 milligrammes du composé par kg chez les mammifères.

17. Utilisation selon l'une des revendications 1 à 16, **caractérisée en ce que** le composé est utilisé en association avec un autre composé.

## Patentansprüche

1. Verwendung einer Verbindung der nachfolgenden Formeln I, II, III oder IV 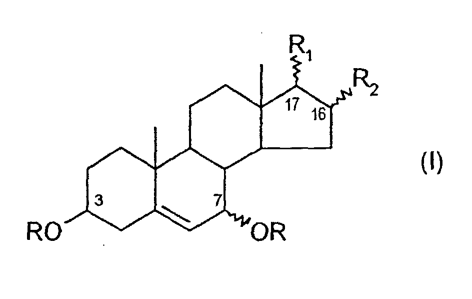 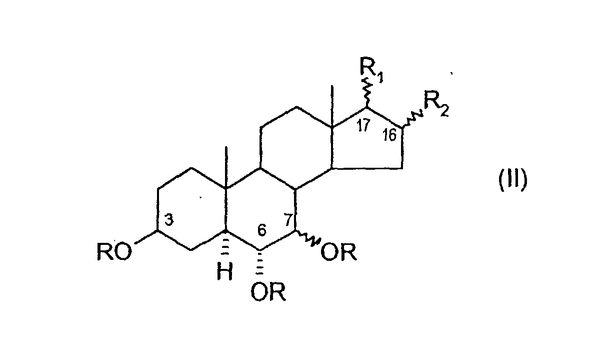 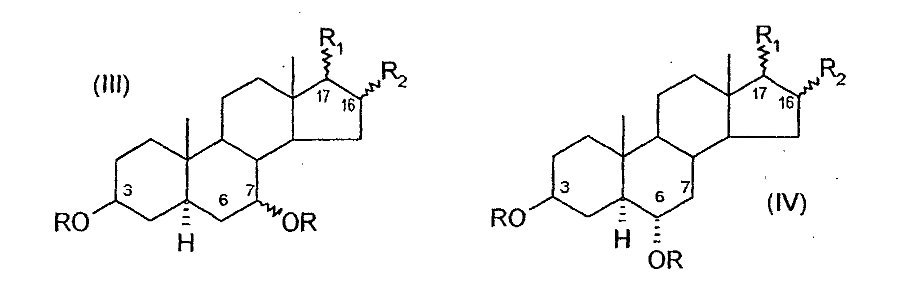 und in welchen:
R = -H oder eine Esterfunktion mit 1 bis 100 Kohlenstoffatomen,
R₁ = -H₂ oder =0 (Keton),
oder β-CO-R₃,
oder β-CHOH-R₃,
oder β-OH,
wobei R₃ eine Alkylgruppe mit 1 bis 10 Kohlenstoffatomen, vorzugsweise Methyl ist, substituiert oder unsubstituiert,
R₂ = -H₂ oder ein Halogen oder ein Carbonitrilrest,
wobei sich die Reste an 6, 7 und 16 in α- oder β-Stellung befinden können, und in welchen gegebenenfalls der Substituent für die Wasserstoffatome an Position 7 ein Keton sein kann, zur Herstellung einer pharmazeutischen Zusammensetzung zur Behandlung von mit Glucocorticoiden assoziierten Pathologien und von unerwünschten Nebenwirkungen, die auf Glucocorticoide zurückzuführen sind, wobei diese Pathologien und Nebenwirkungen ausgewählt sind aus der Gruppe, die besteht aus:
- Cushing Syndrom,
- Nebennierentumoren,
- diabetogenen und lipidogenen Wirkungen von Glucocorticoiden,
- Muskelatrophie und Schwäche,
- Geschwüren des Verdauungstrakts,
- Osteoporose,
- Knochenbrüchigkeit,
- Katarakt,
- Wachstumsverminderung von Kindern,
- Insomnien,
- depressiven Zuständen,
- Dysmenorrhöen,
wobei die Verbindungen I, II, III und IV in der Zusammensetzung vereint mit einem pharmazeutisch verträglichen Trägerstoff vorliegen.

2. Verwendung gemäß Anspruch 1, **dadurch gekennzeichnet, daß** R = H.

3. Verwendung gemäß Anspruch 1 oder 2, **dadurch gekennzeichnet, daß** R₂ = H₂.

4. Verwendung gemäß Anspruch 1 **dadurch gekennzeichnet, daß** R₁ = = β-CO-CH₃.

5. Verwendung gemäß Anspruch 1, **dadurch gekennzeichnet, daß** die Verbindung III 7α-Hydroxy-iso-androsteron ist.

6. Verwendung einer Verbindung gemäß Formel I, II, III oder IV, wie definiert in wenigstens einem der Ansprüche 1 bis 5, zur Herstellung einer pharmazeutischen Zusammensetzung zur Behandlung von pathologischen Manifestationen des Cushing Syndroms oder der Nebennierentumoren.

7. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von diabetogenen und lipidogenen Wirkungen, die auf Glucocorticoide zurückzuführen sind.

8. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von durch Glucocorticoide ausgelöster Muskelatrophie und Schwäche.

9. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von Geschwüren des Verdauungstrakts, ausgelöst durch die Verwendung von oder durch ein Übermaß an Glucocorticoiden.

10. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von Osteoporose und Knochenbrüchigkeit als Folge der Wirkung natürlicher oder synthetischer Glucocorticoide.

11. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von Katarakt als Folge der Wirkung natürlicher oder synthetischer Glucocorticoide.

12. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung der Wachstumsverminderung bei mit Glucocorticoiden behandelten Kindern.

13. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung und Behandlung von Insomnien und depressiven Zuständen als Folge der Wirkung natürlicher oder synthetischer Glucocorticoide.

14. Verwendung einer Verbindung gemäß Formel I, II, III oder IV, wie definiert in wenigstens einem der Ansprüche 1 bis 5, zur Herstellung einer pharmazeutischen Zusammensetzung zur Vorbeugung von Dysmenorrhöen als Folge der Wirkung von Glucocorticoiden.

15. Verwendung gemäß den Ansprüchen 1 bis 14, **dadurch gekennzeichnet, daß** die Verbindung und der pharmazeutische Trägerstoff in einer galenischen Form vereint sind, die eine perorale, perkutane oder topische Verabreichung erlaubt.

16. Verwendung gemäß den Ansprüchen 1 bis 15, **dadurch gekennzeichnet, daß** die Verbindung derart dosiert ist, daß sie bei Säugern eine Verabreichung von 1 bis 100 Milligramm der Verbindung pro kg erlaubt.

17. Verwendung gemäß einem der Ansprüche 1 bis 16, **dadurch gekennzeichnet, daß** die Verbindung in Assoziation mit einer anderen Verbindung verwendet wird.

## Claims

1. Use of a compound of formula I, II, III or IV below: 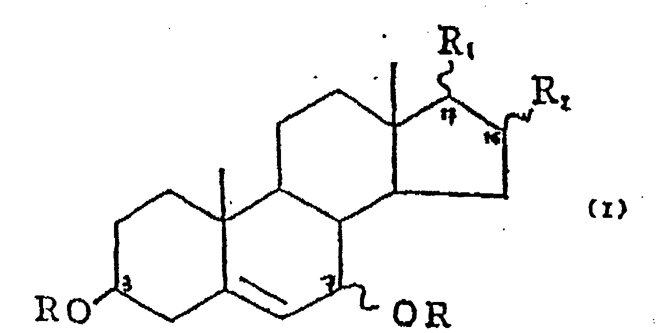 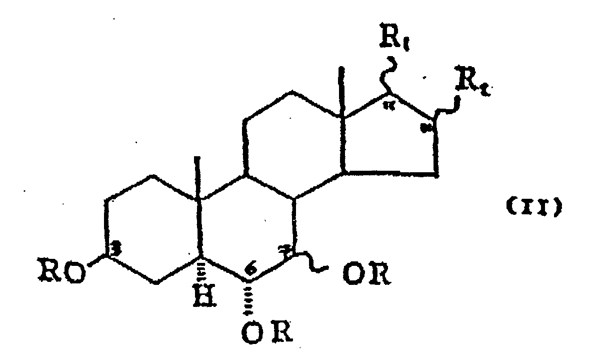 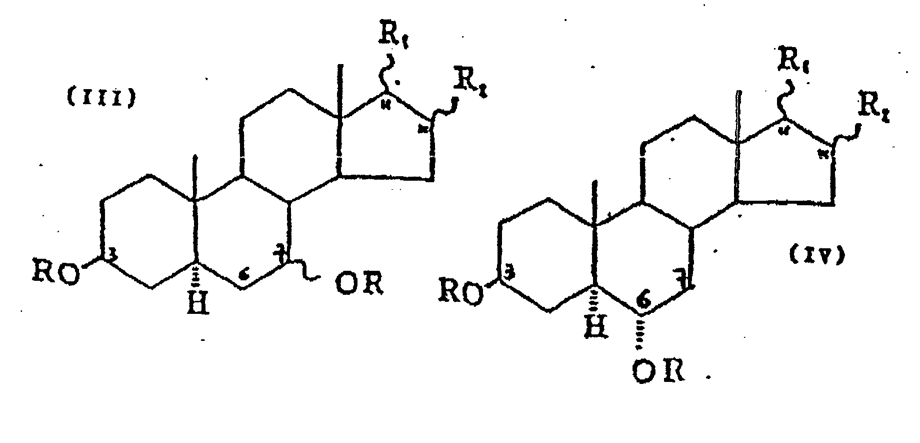 wherein:
R = -H or an ester function with 1 to 100 carbon atoms,
R₁ -H₂ or =O(ketone),
or β-CO-R₃,
or β-CHOH-R₃,
or β-OH,
R₃ is a substituted or unsubstituted alkyl group comprising 1 to 10 carbon atoms, preferably methyl,
R₂ = -H₂ or a halogen, or a carbonitrile radical,
wherein the radicals at 6, 7 end 16 may be in the α or β position, and wherein, if applicable, the substituent for the hydrogens in position 7 may be a ketone,
in the production of a pharmaceutical composition for treating pathological conditions associated with glucocorticoids and the undesirable side effects caused by glucocorticoids, these pathological conditions and side effects being selected from the list comprising:
- Cushing's syndrome,
- suprarenal tumours,
- diabetogenic and lipidogenic effects of glucocorticoids,
- muscular atrophy and weakness,
- digestive ulcers,
- osteoporosis,
- brittle bones,
- cataract,
- diminished growth in children,
- insomnia,
- depression,
- dysmenorrhoea,
compounds I, II, III and IV being combined in said composition with a pharmaceutically acceptable carrier.

2. Use according to claim 1, **characterised in that** R = H.

3. Use according to claim 1 or 2, **characterised in that** R₂ = H₂.

4. Use according to claim 1, **characterised in that** R₁ = β-CO-CH₃.

5. Use according to claim 1, **characterised in that** compound III is 7α-hydroxy-iso-androsterone.

6. Use of a compound of formula I, II, III or IV as defined in at least one of claims 1 to 5, for preparing a pharmaceutical composition for treating pathological symptoms of Cushing's syndrome or suprarenal tumours.

7. Use of a compound of formula I, II, III or IV as defined in at least one of claims 1 to 5, for preparing a pharmaceutical composition for the prevention and treatment of diabetogenic and lipidogenic effects caused by glucocorticoids.

8. Use of a compound of formula I, II, III or IV as defined in at least one of claims 1 to 5, for preparing a pharmaceutical composition for the prevention and treatment of muscular atrophy and weakness caused by glucocorticoids.

9. Use of a compound of formula I, II, III or IV as defined in at least one of claims 1 to 5, for preparing a pharmaceutical composition for the prevention and treatment of digestive ulcers caused by the use of or an excess of glucocorticoids.

10. Use of a compound of formula I, II, III or IV as defined in at least one of claims 1 to 5, for preparing a pharmaceutical composition for the prevention and treatment of osteoporosis and brittle bones resulting from the action of natural or synthetic glucocorticoids.

11. Use of a compound of formula I, II, III or IV as defined in at least one of claims 1 to 5, for preparing a pharmaceutical composition for the prevention and treatment of cataract resulting from the action of natural or synthetic glucocorticoids .

12. Use of a compound of formula I, II, III or IV as defined in at least one of claims 1 to 5, for preparing a pharmaceutical composition for the prevention of diminished growth in children treated with glucocorticoids.

13. Use of a compound of formula I, II, III or IV as defined in at least one of claims 1 to 5, for preparing a pharmaceutical composition for the prevention and treatment of insomnia and depression resulting from the action of natural or synthetic glucocorticoids.

14. Use of a compound of formula I, II, III or IV as defined in at least one of claims 1 to 5, for preparing a pharmaceutical composition for the prevention of dysmenorrhoea resulting from the action of glucocorticoids.

15. Use according to claims 1 to 14, **characterised in that** the compound and the pharmaceutical carrier are combined in a galenic form allowing them to be administered either by oral route or transcutaneously or topically.

16. Use according to claims 1 to 15, **characterised in that** the compound is metered to allow the administration of 1 to 100 milligrams of the compound per kg to mammals.

17. Use according to claims 1 to 16, **characterised in that** the compound is used in combination with another compound.
